# DEMANDE DE BREVET EUROPEEN

(11) **EP 4 714 932 A1**
(43) Date de publication de la demande: **25.03.2026**
(21) Numéro de dépôt: 25186733.9
(22) Date de dépôt: 01.07.2025
(51) Int. Cl.: C07B 59/00, C07D 307/46

(54) **CANNALACTONE MARQUEE AU DEUTERIUM, SYNTHESE ET UTILISATION POUR LE DOSAGE DE LA CANNALACTONE DANS TOUS TISSUS OU EXSUDATS DE PLANTES OU ORGANISMES VIVANTS**

(30) Priorité: 01.07.2024 FR 2407158
(71) Demandeur: Centre National de la Recherche Scientifique (CNRS), 75016 Paris (FR); Université Paris-Saclay, 91190 Gif-sur-Yvette (FR); Nantes Université, 44035 Nantes Cedex 1 (FR); Hemp it Adn, 49250 Beaufort-en-Anjou (FR)
(72) Inventeur: BOYER, François Didier, 78150 Le Chesnay-Rocquencourt (FR); POUVREAU, Jean Bernard, 44390 Nort sur Erdre (FR); MACIUK, Alexandre, 92290 Châtenay Malabry (FR); DAIGNAN FORNIER DE LACHAUX, Suzanne, 91300 Massy (FR); MATHIS, Fabienne, 49100 Angers (FR)
(74) Mandataire: Oak & Fox

(57) **Abrégé**

La présente invention concerne la synthèse chimique de la cannalactone, une adaptation de cette synthèse conduisant à un marquage au deutérium de la cannalactone, ainsi que l'utilisation de la cannalactone marquée au deutérium pour effectuer le dosage de la cannalactone naturelle des exsudats ou tissus de la plante de chanvre.

## Description

### DOMAINE TECHNIQUE DE L'INVENTION

La présente invention concerne la synthèse chimique de la cannalactone, une adaptation de cette synthèse conduisant à un marquage au deutérium de la cannalactone, ainsi que l'utilisation de la cannalactone marquée au deutérium pour effectuer le dosage de la cannalactone dans tous tissus ou exsudats de plantes ou organismes vivants, et notamment dans les exsudats ou tissus de la plante de chanvre par chromatographie liquide couplée à la spectrométrie de masse.

### Arrière-plan technique

Le chanvre (*Cannabis sativa*) est une plante annuelle originaire d'Asie exploitée depuis plus de 8 000 ans. Elle est cultivée dans le monde entier et est capable de couvrir les quatre besoins vitaux de l'humanité : se nourrir, se loger, s'habiller, se soigner.

La culture de chanvre est répandue car il s'agit d'une production rentable et durable. En effet, la plante s'adapte facilement aux terrains et aux climats divers et pour cela ne nécessite aucun traitement phytosanitaire. La surface de production de chanvre en France a été multipliée par trente entre 1960 et aujourd'hui. Cet intérêt pour le chanvre ne fait que croître, en partie dans le but de remplacer peu à peu le coton, très demandeur en eau. La France est aujourd'hui le premier producteur de chanvre en Europe avec une production sur environ 20 000 ha.

Connue pour sa résistance aux parasites et aux ravageurs, la plante de chanvre en est d'autant plus attractive. Cependant une plante parasite de la famille des orobanches, l'orobanche rameuse, *Phelipanche ramosa* induit de grandes pertes de rendement sur les cultures de chanvre pouvant aller jusqu'à plus de 80 %**.** Ce parasite s'attaque aussi à des cultures telles que le colza ou le tabac mais une spécialisation d'une population d'orobanche rameuse au chanvre a été mise en évidence [1]-[3].

Après germination, la plante parasite se connecte à la racine de la plante hôte et récupère ainsi les nutriments pour son propre développement [4]. Ce parasitisme crée des dommages importants sur les cultures de chanvre.

Les strigolactones sont des petites molécules connues comme étant exsudées dans le sol à des concentrations picomolaires (10⁻¹² M) et ont été identifiées comme stimulants de germination des graines de Phelipanche ramosa ou orobanche rameuse [5], [6]. Les strigolactones (SL) ont été identifiées pour la première fois pour leur rôle dans les interactions parasitaires et symbiotiques dans la rhizosphère et constituent la dernière classe d'hormones végétales à avoir été découverte [7], [8] Elles sont surtout connues pour leur rôle dans le contrôle de l'architecture des plantes, plus récemment, des rôles pour les SL dans d'autres aspects du développement des plantes ont été mise en évidence [9].

Le Demandeur a identifié que la cannalactone, qui est une strigolactone exsudée par le chanvre, était responsable de l'initiation du cycle de vie parasitaire de l'orobanche rameuse [1], [10], [11].

A cette fin, il a développé une synthèse chimique permettant l'accès à la cannalactone (faiblement biodisponible), ainsi qu'une adaptation de cette synthèse pour l'obtention d'une cannalactone marquée au deutérium et son utilisation pour effectuer le dosage de la cannalactone naturelle des exsudats ou tissus de la plante de chanvre.

### Résumé de l'invention

En particulier, la présente invention a pour objet la cannalactone marquée au deutérium, caractérisée en ce qu'elle répond à la formule générale 1 : 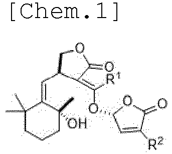 dans laquelle :
- R¹ désigne un atome d'hydrogène H ou un atome de deutérium D,
- R²désigne le radical méthyle CH₃ ou le radical méthyle deutéré tri-substitué CD₃,
- l'un au moins de R¹ et R² étant deutéré.

De manière avantageuse, R¹ peut désigner un atome d'hydrogène H et R² peut désigner le radical méthyle deutéré tri-substitué CD₃, de sorte que la cannalactone marquée au deutérium selon l'invention répondra à la formule 2 suivante :

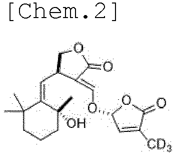

De manière avantageuse, R¹ peut désigner un atome de deutérium D et le R² peut désigner le radical méthyle CH₃, de sorte que la cannalactone marquée au deutérium selon l'invention répondra à la formule 3 suivante :

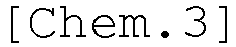

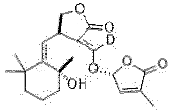

De manière avantageuse, R¹ peut désigner un atome de deutérium D et le R² peut le radical méthyle deutéré tri-substitué CD₃, de sorte que la cannalactone marquée au deutérium selon l'invention répondra à la formule 4 suivante :

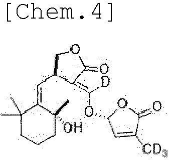

La présente invention a également pour objet un procédé de synthèse de la cannalactone, de formule générale 5 suivante : 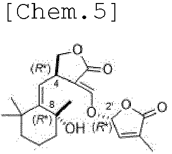 caractérisé en ce qu'il comprend les étapes suivantes :
- une réaction A) de couplage du β-cyclocitral commercial avec un bromofurane C4 de formule 6 suivante : 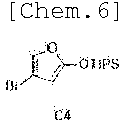 pour obtenir un alcool B20 de formule 7 suivante : [0027] [Chem.7] 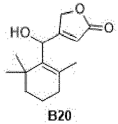
- une étape B) de réduction de l'alcool B20 de formule 7 notamment par un hydrure, pour obtenir un mélange de diastéréoisomères de l'alcool allylique, suivie d'une étape de séparation (notamment par chromatographie sur silice) desdits diastéréoisomères pour retenir le diastéréoisomère (4*R**, 6*R**)-B21 de formule 8 suivante : 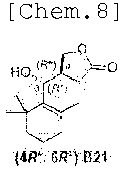
- une étape C) d'époxydation sélective du composé (4*R**, 6*R**)-B21 de formule 8, notamment dirigée par l'alcool allylique, pour obtenir l'époxyde (4*R**, 6*R**)*-cis-*B22 de formule 9 suivante : 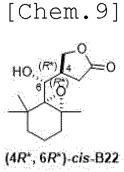
- une réaction radicalaire D) de réduction et d'isomérisation de l'époxyde (4*R**, 6 *R**)*-cis-*B22 de formule 9, pour obtenir l'alcool tertiaire (4*R**, 8*R**)-B1 de formule 10 suivante : 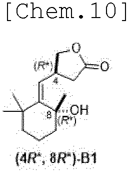
- une étape E) de protection de l'alcool tertiaire (4*R**, 8*R**)- B1 de formule 10, pour obtenir l'alcool protégé (4*R**, 8*R**)-B23 de formule 11 suivante : 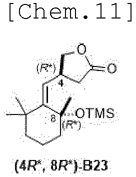
- une étape F) comprenant la formylation F1) du composé (4*R**, 8*R**)-B23 de formule 11 à l'aide de formiate d'alkyle (notamment un formiate d'éthyle ou de méthyle) de formule 12 suivante : 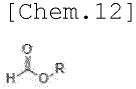
   puis l'*O*-alkylation F2) de l'aldéhyde ainsi formé à l'aide du composé D4 de formule 13 suivante : 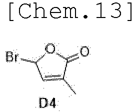
   conduisant à l'obtention du mélange de diastéréoisomères (*4R**, *6R**)-B24 de formule 14 suivante : 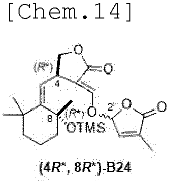
- une étape G) de déprotection de l'alcool tertiaire et de séparation (notamment par chromatographie sur silice) des diastéréoisomères de formule 14, conduisant à l'isolement de la cannalactone de formule 5.

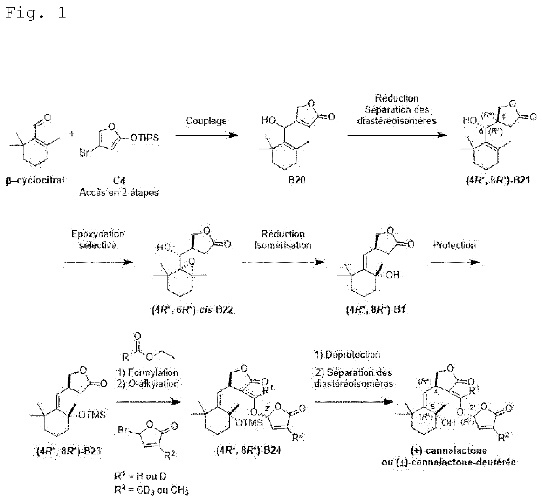

Le procédé de synthèse de la cannalactone selon l'invention est illustré ci-après par la [Fig.1] qui montre la voie d'accès en dix étapes mise au point par les inventeurs pour synthétiser chimiquement la cannalctone.

La présente invention a aussi pour objet un procédé de synthèse d'une cannalactone marquée au deutérium selon l'invention décrite précédemment caractérisée en ce qu'il comprend les étapes suivantes :
- les étapes A à E de synthèse du composé protégé (4*R**, 8*R**)-B23 de formule 11 telles que décrites précédemment pour la synthèse de la cannalactone non deutérée (les étapes A à E sont strictement identiques) ;
- une étape F) comprenant la formylation F1) du composé (4*R**, 8*R**)-B23 de formule 11 à l'aide du formiate d'alkyle (notamment un formiate d'éthyle ou de méthyle) répondant à la formule 12
   ou
   à l'aide du formiate d'alkyle deutéré (notamment un formiate d'éthyle ou de méthyle deutéré) répondant à la formule 15 suivante : 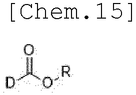
- puis l'*O*-alkylation de l'aldéhyde ainsi formé à l'aide du composé D4 (notamment celui décrit dans la publication [12]) de formule 13 suivante : 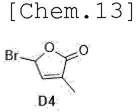 ou à l'aide du composé D4 deutéré (notamment celui décrit dans la publication [0053][13]) de formule 16 suivante : 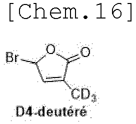
   l'un au moins du composé organique fonctionnalisé par un groupe formyle ou du composé D4 étant deutéré,
   pour obtenir un mélange de diastéréoisomères deutérés (4*R**, 6*R**)-B24 de formule 17 suivante : 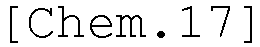 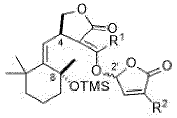 avec
- R¹ désignant un atome d'hydrogène H ou un atome de deutérium D, et
- R²désigne un groupement méthyle (CH₃) ou un groupement méthyle où les hydrogènes ont été remplacés par des atomes de deutérium (CD₃),
- l'un au moins de R¹ et R² étant deutéré ;
- une étape G) de déprotection de l'alcool tertiaire et de séparation des diastéréoisomères (notamment par chromatographie sur silice), conduisant à l'isolement de la cannalactone marquée au deutérium de formule 1.

Le procédé de synthèse de la cannalactone marquée au deutérium selon l'invention est illustré ci-après également par la Figure 1.

De manière avantageuse, selon un premier mode de réalisation avantageux du procédé de synthèse d'une cannalactone marquée au deutérium, la formylation F1) peut être réalisée à l'aide du formiate d'alkyle (notamment du formiate d'éthyle ou du formiate de méthyle) répondant à la formule 12, et l'O-alkylation F2) de l'aldéhyde à l'issue de la formylation F1) peut être réalisée à l'aide du composé D4 de formule 16. Dans ce cas, ce mode de réalisation conduira à la formation d'une cannalactone marquée au deutérium répondant à la formule 2.

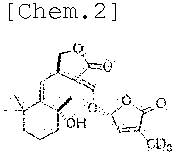

De manière avantageuse, selon un deuxième mode de réalisation avantageux du procédé de synthèse d'une cannalactone marquée au deutérium, la formylation F1) peut être réalisée à l'aide du formiate d'alkyle (notamment du formiate d'éthyle ou du formiate de méthyle) répondant à la formule 15, et l'*O*-alkylation F2) de l'aldéhyde à l'issue de la formylation F1) est réalisée à l'aide du composé D4 de formule 13. Dans ce cas, ce mode de réalisation conduira à la formation d'une cannalactone marquée au deutérium répondant à la formule 3.

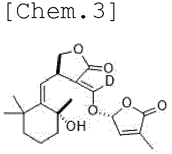

De manière avantageuse, selon un troisième mode de réalisation avantageux du procédé de synthèse d'une cannalactone marquée au deutérium, la formylation F1) peut être réalisée à l'aide du formiate d'alkyle deutéré (notamment du formiate de méthyle deutéré ou formiate d'éthyle deutéré) répondant à la formule 15, et l'*O* - alkylation F2) de l'aldéhyde à l'issue de la formylation F1) est réalisée à l'aide du composé D4 de formule 16. Dans ce cas, ce mode de réalisation conduira à la formation d'une cannalactone marquée au deutérium répondant à la formule 4.

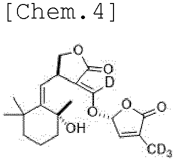

Enfin, la présente invention concerne en outre l'utilisation de la cannalactone marquée au deutérium selon l'invention ou telle qu'obtenue selon le procédé selon l'invention relatif à l'obtention de de la cannalactone marquée au deutérium, pour effectuer le dosage de la cannalactone dans tous tissus ou exsudats de plantes ou organismes vivants, et notamment dans les exsudats ou tissus de la plante de chanvre, et en particulier pour effectuer le dosage de la cannalactone naturelle des exsudats ou tissus de la plante de chanvre par chromatographie liquide couplée à la spectrométrie de masse.

### Brève description des figures

D'autres caractéristiques et avantages de l'invention pourront encore apparaître à l'homme du métier à la lecture des exemples ci-dessous, donnés à titre illustratif et non limitatif et illustrés par la [Fig.1] annexée:

La figure 1 représente le schéma de la synthèse totale racémique de la cannalactone marquée ou non au deutérium.

### EXEMPLES

### Solvants et réactifs

Les réactifs chimiques, sont des produits commerciaux notamment commercialisés par les sociétés Sigma Aldrich, Alfa Aesar, Acros Organics et TCI. Ils ont été utilisés sans purification supplémentaire.

Les solvants secs de qualité analytique sont des produits commerciaux notamment commercialisés par les sociétés Sigma Aldrich et Acros Organics. Le tétrahydrofurane (THF) a été distillé sous argon sur sodium en présence de benzophénone. Les solvants deutérés sont commercialisés par la société Eurisotop.

### Matériels et Méthodes

Les réactions non aqueuses ont été effectuées sous atmosphère inerte (argon ou azote), en utilisant les techniques standards de manipulation des composés sensibles à l'air et à l'humidité.

Toutes réactions ont été suivies par chromatographie sur couche mince (CCM) sur des plaques d'aluminium pré-enduites de gel de silice (commercialisé par la société Merck sous la dénomination commerciale 60 F254 avec détection par UV à courte longueur d'onde (c'est-à-dire λ = 254 nm), et/ou par coloration avec une solution de KMnO₄ [1 % (p/p)] dans de l'eau ou une solution de vanilline [1 % (p/p)] dans une solution éthanoïque à 1 % (v/v) d'acide phosphorique.

La plupart des séparations ont été effectuées dans des conditions de Flashchromatographie sur gel de silice à l'aide d'une cartouche garnie (gel de silice de 40-63 µm) à pression moyenne (20 psi) avec un collecteur de fraction Armen ou une pompe ou un Buchi Pure C-805 Flash.

Certaines séparations ont été réalisées sur chromatographie préparative en couche mince (PTLC) (gel de silice Merck 60 F254 sur verre).

Les spectres RMN ¹H ont été enregistrées sur des spectromètres Bruker à 300, 500 ou 700 MHz. Les spectres RMN ¹³C ont été enregistrées sur les mêmes instruments à 75, 125 ou 175 MHz. Les déplacements chimiques δ sont exprimés en parties par million (ppm) avec les signaux des solvants résiduels comme référence interne (δ = 7,24 pour la RMN ¹H et 77,23 pour la RMN ¹³C dans CDCl₃). Pour la RMN ¹H, les spectres sont décrits comme suit : déplacement chimique, intégration, multiplicité (s = singulet, d = doublet, t = triplet, q = quadruplet, quint = quintuplet, sext = sextuplet, dd = doublet de doublet, dt = doublet de triplet, m = multiplet), constante de couplage en Herz (J) et attribution. Toutes les attributions RMN sont basées sur des expériences COSY, HSQC et HMBC. Des expériences NOESY ont été enregistrées pour confirmer les configurations des doubles liaisons.

Les spectres IR ont été enregistrés sur un spectromètre PerkinElmer Spectrum 100 FT-IR, les absorptions étant données en centimètres⁻¹ (cm⁻¹).

Les spectres de masse à basse résolution ont été déterminés par ionisation par électronébulisation sur un système UPLC Acquity de Waters, combiné à un détecteur à photodiodes (PDA), un détecteur de diffusion de la lumière par évaporation (ELSD) et par un spectromètre de masse avec un détecteur quadripolaire en tandem (TQD). Les tampons et les phases mobiles aqueuses pour l'UPLC ont été préparés en utilisant de l'eau purifiée avec un système Milli-Q.

Les spectres de masse à haute résolution ont été obtenus avec le dispositif UPLC Waters Acquity (par injection directe ou avec une colonne BEH C₁₈ 2,1 Å ~50 mm, 1,7 µm) combiné à un PDA et un instrument de masse Waters LCT Premier XE (ESI avec un analyseur de temps de vol (ToF)).

Les mesures du pouvoir rotatoire ont été enregistrées sur un polarimètre Anton Paar MCP 300 utilisant une cellule de 1 dm de long.

Les mesures de dichroïsme circulaire ont été enregistrées sur un spectropolarimètre JASCO J-810 à une vitesse de balayage de 50 nm/min dans l'acétonitrile de 200 à 400 nm.

La séparation chirale d'énantiomères synthétiques de cannalactone a été réalisée sur un appareil à fluide supercritique THAR. Cet instrument, commercialisé sous la dénommination "Investigator II", est composé d'un module de pompe pour le CO₂ainsi que pour le co-solvant, d'un passeur d'échantillons, d'un régulateur de pression et d'un collecteur pouvant contenir jusqu'à 6 fractions.

La détection a été effectuée à l'aide d'un spectromètre UV à bande d'iode (commercialisé sous la dénomination commerciale Waters PDA 2998n de 200 à 800 nm). Les paramètres de l'instrument ont été optimisés pour la séparation du mélange racémique. La colonne utilisée est une colonne IC (silice immobilisée à base de cellulose protégée par du tris(3,5-dichlorophénylcarbamate)) de Daicel, de taille 4,6 x 250 mm et de granulométrie 5 µm. La température du four a été fixée à 25 °C et la pression appliquée au système était de 100 bars. Le débit total était fixé à 4 mL/min, avec 10 % (v/v) de méthanol. Les énantiomères ont été séparés dans deux flacons à l'aide d'une pompe d'appoint à un débit fixe de 3 mL/min de méthanol. Les conditions de collecte sont résumées dans le tableau 1 suivant :

**Tableau 1**

| | Longueur d'on de (nm) | Seuil Début (m Au) | Seuil Arrêt (mA u) | Fenêtre de te mps Début (min) | Fenêtre de temp s Arrêt (min) |
|---|---|---|---|---|---|
| Fiole 1 | 226 | 20 | 30 | 22, 8 | 27,5 |
| Fiole 2 | 226 | 20 | 20 | 22.8 | 33 |

### EXEMPLE 1 : synthèse de la cannalactone naturelle conformément au premier procédé selon l'invention (voie d'accès illustrée par la figure 1)

### 4-Bromofuran-2(5H)-one (C8)

A une solution de furan-2,4(3*H*,5*H*)-dione (1,0 g, 10,00 mmol) dans du CH₂Cl₂ (22 mL) et du DMF (1 mL) à 0 °C on a ajouté du dibromure d'oxalyle (2,6 g, 12,00 mmol, 1,2 équiv.). Le mélange a été agité pendant 1 h à 0 °C et réchauffé progressivement à température ambiante pendant 2 h. Le mélange réactionnel a été dilué avec de l'eau (50 mL) et extrait avec de l'EtOAc (3 x 20 mL). Les phases organiques combinées ont été lavées avec de l'eau (2 x 30 mL), une solution aqueuse de NaHCO₃ saturée (2 x 30 mL) et de la saumure (2 x 30 mL) et séchées sur Na₂SO₄. Les solvants ont été éliminés pour obtenir le produit brut C8 (1,61 g, quantitatif) sous la forme d'un solide brun. Les analyses chimiques sont en accord avec la littérature [14].

### (4-Bromofuran-2-yl)oxytriisopropylsilane (C4)

A une solution de 4-bromofuran-2(5*H*)-one (C8) (720,4 mg, 4,40 mmol) dans du CH₂Cl₂ (6,2 mL) sous argon à 0 °C, on a ajouté de la Et₃N (626,4 mg, 6,20 mmol, 1,4 équiv.). Le mélange a été agité pendant 1 minute, puis du trifluorométhanesulfonate de triisopropylsilyle (TIPSOTf) (1,42 g, 4,60 mmol, 1,05 équiv.) a été ajouté goutte à goutte à 0 °C. Le mélange ainsi obtenu a été agité pendant 10 minutes à 0 °C, puis réchauffé à la température ambiante et agité pendant encore 1 h 30. Le mélange a été dilué avec de l'heptane (10 mL), lavé avec une solution aqueuse de NaHCO₃ saturée (2 x 10 mL), de l'eau (2 x 10 mL) et de la saumure (2 x 10 mL). La phase organique a été séchée sur Na₂SO₄. Les solvants ont été éliminés pour obtenir le bromofurane C4 (1,4 g, quantitatif) sous forme d'huile brune. Les analyses chimiques sont en accord avec la littérature [14].

### 4-[Hydroxy(8,12,12-triméthylcyclohex-7-en-6-yl)méthyl]furan-2(5 H )-one (B20)

A une solution de C4 (89,9 mg, 0,28 mmol) dans du THF sec (1,8 mL) sous argon à -78 °C, une solution de *n*-BuLi est ajoutée goutte à goutte (0,3 mL, 0,30 mmol, 0,98 M, 1,1 équiv.). Le mélange ainsi obtenu a été agité à -78 °C pendant 30 minutes. Un mélange de β-cyclocitral (51,6 mg, 0,34 mmol, 1,2 équiv.) dans du THF sec (2 mL) y est ensuite ajouté. Le mélange réactionnel a été agité pendant 2 h à -78 °C et 12 h à température ambiante. Le mélange a été hydrolysé avec une solution aqueuse de NH 4Cl saturée (5 mL) et une solution aqueuse de HCl (5 mL, 2 M). La phase organique a été séparée et la phase aqueuse a été extraite avec EtOAc (3 x 5 mL). Les phases organiques combinées ont été lavées avec de l'eau (2 x 5 mL), une solution aqueuse de NaHCO₃ saturée (2 x 5 mL), de l'eau (2 x 5 mL) et de la saumure (2 x 5 mL), puis séchées sur Na₂SO₄. Les solvants ont été éliminés et le mélange a été purifié par chromatographie sur gel de silice (heptane/EtOAc, 95:5 à 60:40 pendant 20 min) pour obtenir le produit pur B20 (24,5 mg, 37%) sous forme d'huile brune, de formule 7 :

### B20

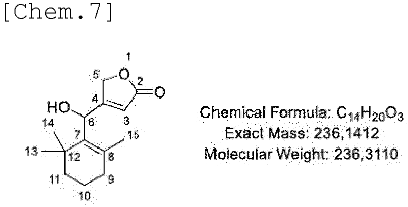

RMN ¹H (500 MHz, CDCl₃) δ 5.91 (1H, d, J = 1.5 Hz, H-3), 5.10 (1H, s, H-6), 4.88 (1H, d, J = 18.0 Hz, H-5a), 4.71 (1H, d, J = 18.0 Hz, H-5b), 1.96 (2H, t, J = 6.0 Hz, H-9), 1.61 (3H, s, H-15), 1.59-1.55 (2H, m, H-10), 1.50-1.46 (2H, m, H-11), 1.13 (3H, s, H-13 ou H-14), 0.98 (3H, s, H-13 ou H-14).

RMN ¹³C (75 MHz, CDCl₃) δ 174.1 (C-2), 174.0 (C-4), 138.7 (C-7), 136.6 (C-8), 115.0 (C-3), 71.9 (C-5), 67.8 (C-6), 39.5 (C-11), 35.0 (C-12), 33.7 (C-9), 28.9 (C-13 ou C-14), 28.5 (C-13 ou C-14), 21.4 (C-15), 19.3 (C-10).

IR (film) νₘₐₓ 3471, 2932, 1777, 1741, 1637, 1447, 1268, 1111, 1028 cm⁻¹.

HRESIMS *m*/*z* 237.1491 [M + H]⁺ (calc. pour C₁₄H₂₁O_{3,} 237.1491).

### 4-[Hydroxy(8,12,12-triméthylcyclohex-7-èn-6-yl)méthyl]dihydrofuran-2(3 H )one (B21)

A une solution de B20 (696.4 mg, 2.95 mmol) dans du méthanol (45 mL) à 15 °C, est ajouté du NiCl₂ (350.9 mg, 1.48 mmol, 0.5 équiv.) puis du borohydrure de sodium (358,3 mg, 9,47 mmol, 3,2 équiv.) par portions. Le mélange a été agité à 15 °C jusqu'à ce que l'analyse TLC indique une conversion complète. Le mélange réactionnel a été hydrolysé avec une solution aqueuse de HCl (50 ml, 2 M). La phase aqueuse a été extraite avec CH₂Cl₂ (3 x 20 mL). Les phases organiques combinées ont été séchées sur Na₂SO₄ et les solvants ont été éliminés. Le mélange obtenu a ensuite été purifié par chromatographie sur gel de silice (CH₂Cl₂/EtOAc, 100:0 à 90:10 pendant 30 min) pour obtenir le produit pur (4*R**, 6*R**)-B21 de formule 8 (327,4 mg, 47%) sous forme d'huile jaune et (4*R**, 6*S**)-B21 de formule 18 (131,9 mg, 19%) sous forme de solide blanc :

### (4R*, 6R*)-B21

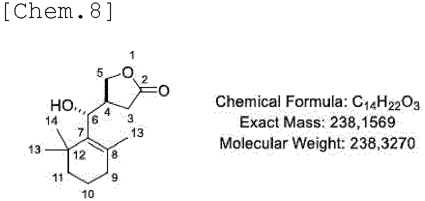

RMN ¹H (500 MHz, CDCl₃) δ 4.50 (1H, dd, J = 9.5, 7.0 Hz, H-5a), 4.28 (1H, dd, J = 9.5, 7.0 Hz, H-5b), 4.21 (1H, d, J = 9.5 Hz, H-6), 3.21 (1H, sext, J = 9.5 Hz, H-4), 2.41 (1H, dd, J = 17.5, 8.5 Hz, H-3a), 2.18 (1H, dd, J = 17.5, 8.5 Hz, H-3b), 1.96 (2H, q, J = 5.5 Hz, H-9), 1.81 (3H, s, H-15), 1.59-1.52 (2H, m, H-10), 1.48-1.45 (1H, m, H-11a), 1.40-1.35 (1H, m, H-11b), 1.08 (3H, s, H-13 ou H-14), 0.98 (3H, s, H-13 ou H-14).

RMN ¹³C (125 MHz, CDCl₃) δ 177.1 (C-2), 138.4 (C-7), 135.1 (C-8), 72.9 (C-5), 72.6 (C-6), 41.3 (C-4), 40.4(C-11), 35.0 (C-12), 34.6 (C-9), 32.4 (C-3), 29.2 (C-13 ou C-14), 29.1 (C-13 ou C-14), 21.3 (C-15), 19.4 (C-10).

IR (film) νₘₐₓ 3464, 2928, 1768, 1551, 1365, 1263, 1178, 1048, 1001, 892 cm⁻¹.

HRESIMS *m*/*z* 239.1640 [M + H]⁺ (calc. pour C₁₄H₂₃O₃, 239.1647).

### (4R*, 6S*)-B21

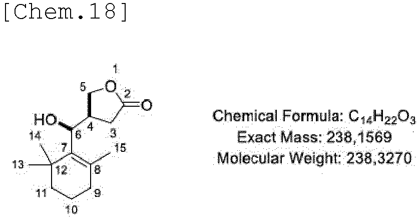

RMN ¹H (500 MHz, CDCl₃) δ 4.17 (1H, dd, J = 9.0, 7.0 Hz, H-5a), 4.16 (1H, d, J = 9.0 Hz, H-6), 3.94 (1H, dd, J = 9.0, 7.0 Hz, H-5b), 3.19 (1H, sext, J = 9.0 Hz, H-4), 2.72 (1H, dd, J = 17.5, 7.5 Hz, H-3a), 2.57 (1H, dd, J = 17.5, 7.5 Hz, H-3b), 1.96 (2H, q, J = 5.0 Hz, H-9), 1.80 (3H, s, H-15), 1.59-1.51 (2H, m, H-10), 1.47-1.44 (1H, m, H-11a), 1.40-1.34 (1H, m, H-11b), 1.08 (3H, s, H-13 ou H-14), 0.97 (3H, s, H-13 ou H-14).

RMN ¹³C (125 MHz, CDCl₃) δ 177.5 (C-2), 138.2 (C-7), 135.1 (C-8), 72.3 (C-6), 70.5 (C-5), 41.5 (C-4), 40.4 (C-11), 35.1 (C-12), 34.6 (C-9), 33.8 (C-3), 29.2 (C-13 ou C-14), 28.8 (C-13 ou C-14), 21.4 (C-15), 19.4 (C-10).

IR (film) νₘₐₓ 3481, 2925, 2870, 1774, 1547, 1465, 1373, 1258, 1176, 1092, 1033, 1011, 890, 795 cm⁻¹.

HRESIMS *m*/*z* 239.1638 [M + H]⁺ (calc. pour C₁₄H₂₃O₃ 239.1647).

### (4 R *)-[(6 R *)-hydroxy(8,12,12-triméthyl-7-oxabicyclo[4.1.0]heptan-6yl)méthyl]dihydrofuran-2(3 H )-one ((4 R *, 6 R *)- cis -B22)

A une solution de (4*R**, 6*R**)-B21 (100.9 mg, 0.420 mmol) dans du toluène sec (5.1 mL), on a ajouté une solution de VO(acac)₂ (3.9 mg, 0.015 mmol, 0.04 équiv.) dans du toluène sec (0.2 mL). Du *tert*-Butyl hydroperoxide (TBHP) (0,11 mL, 5,5 M, 0,590 mmol, 1,4 équiv.) a été ensuite ajouté. Le mélange obtenu a été agité à température ambiante pendant 1 h. Le mélange réactionnel a été hydrolysé avec une solution aqueuse de NaOH (5 mL, 5%). La phase aqueuse a été extraite avec de l'heptane et de l'EtOAc (2:1) (3 x 10 mL). Les phases organiques combinées ont été lavées avec de la saumure (2 x 10 mL), séchées sur Na₂SO₄ et les solvants ont été éliminés pour obtenir le produit pur (4*R**, 6*R**)*-cis-*B22 de formule 9 (116,4 mg, quantitatif) sous forme d'huile incolore utilisée dans l'étape suivante sans purification :

### (4R*, 6R*)-cis-B22

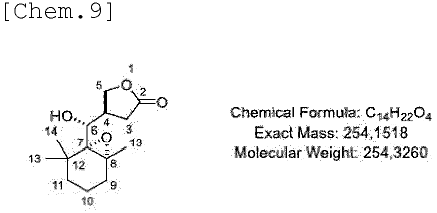

RMN ¹H (300 MHz, CDCl₃) δ 4.40 (1H, dd, J = 9.5, 8.0 Hz, H-5a), 4.31 (1H, dd, J = 9.5, 8.0 Hz, H-5b), 3.96 (1H, d, J = 8.0 Hz, H-6), 2.94 (1H, sext, J = 8.0 Hz, H-4), 2.59 (1H, dd, J = 17.0, 9.0 Hz, H-3a), 2.48 (1H, dd, J = 17.0, 9.0 Hz, H-3b), 1.90-1.80 (1H, m, H-9a), 1.78-1.69 (1H, m, H-9b), 1.39 (3H, s, H-15), 1.36-1.32 (2H, m, H-10), 1.25-1.22 (1H, m, H-11a), 1.06 (3H, s, H-13 ou H-14), 1.05-1.03 (1H, m, H-11b), 1.02 (3H,
s, H-13 ou H-14).

RMN ¹³C (75 MHz, CDCl₃) δ 176.4 (C-2), 71.1 (C-5), 70.5 (C-6), 70.4 (C-7), 66.3 (C-8), 40.0 (C-4), 37.6 (C-11), 33.9 (C-13), 33.3 (C-3), 31.8 (C-9), 25.6 (C-13 et C-14), 22.2 (C-15), 17.0 (C-10).

IR (film) νₘₐₓ 3464, 2928, 1768, 1551, 1365, 1263, 1178, 1048, 1001, 892 cm⁻¹.

HRESIMS *m*/*z* 255.1607 [M + H]⁺ (calc. pour C₁₄H₂₃O₄, 255.1596)

### (4 R *)-( Z )-[(8 R *)-hydroxy-(8,12,12-triméthylcyclohexylidene)méthyl ]dihydrofuran-2(3 H )-one ((4 R *, 8 R *)-B1)

Une solution de dichlorure de titanocène (298,7 mg, 1,20 mmol, 5,0 équiv.) et de manganèse (197,8 mg, 3,60 mmol, 15,0 équiv.) dans du THF sec strictement désoxygéné (1,2 mL) sous argon a été agitée pendant 1 h dans un ballon fermé avec un bouchon Rodavis. La solution est passée du rouge au vert. A ce mélange, on a ajouté une solution de (4*R**, 6*R**)-*cis-*B22 (61,1 mg, 0,24 mmol) dans du THF sec strictement désoxygéné (1,2 mL) sous argon. Le mélange obtenu a été agité pendant 22 heures à température ambiante. Le mélange réactionnel a été hydrolysé avec une solution aqueuse de NaH₂PO₄ saturée (3 mL). La phase aqueuse a été extraite avec de l'EtOAc (3 x 5 mL). La phase organique a été lavée avec une solution aqueuse de NaHCO₃ saturée (2 x 5 mL) et de la saumure (2 x 5 mL), séchée avec Na₂SO₄ et les solvants ont été éliminés. Le produit brut a été purifié par chromatographie sur gel de silice
(heptane/EtOAc, 85:15 à 70:30 pendant 20 min) pour obtenir un mélange de (4*R**, 6*R **)*-cis-*B22, (4*R**, 6*R**)-B21 et (4*R**, 8*R**)-B1 (58,2 mg).

Le mélange est alors dilué dans de la pyridine (0,5 ml) et de l'Ac₂O est ajouté (10 gouttes). Cette réaction a été agitée pendant 48 h à température ambiante jusqu'à ce que l'analyse TLC indique une conversion complète. Le produit brut a été purifié par chromatographie sur gel de silice (heptane/EtOAc, 90:10 à 70:30 pendant 20 min) pour obtenir le produit pur (4*R**, 8*R**)-B1 de formule 10 (11,4 mg, 20%) sous forme d'huile incolore :

### (4R*, 8R*)-B1

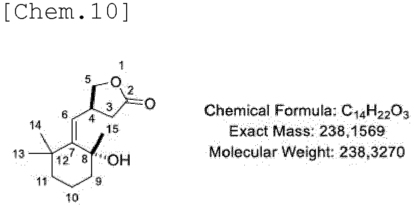

RMN ¹H (500 MHz, CDCl₃) δ 5.25 (1H, d, J = 9.5 Hz, H-6), 4.46 (1H, t, J = 8.0 Hz, H-5a), 4.30 (1H, sext, J = 8.0, H-4), 3.89 (1H, t, J = 8.0 Hz, H-5b), 2.61 (1H, dd, J = 17.5, 9.0 Hz, H-3a), 2.18 (1H, dd, J = 17.5, 9.0 Hz, H-3b), 1.84-1.79 (1H, m, H-9a), 1.59-1.55 (2H, m, H-10), 1.54-1.51 (1H, m, H-9b), 1.42 (3H, s, H-15), 1.39-1.34 (2H, m, H-11), 1.12 (3H, s, H-13 ou H-14), 1.05 (3H, s, H-13 ou H-14).

RMN ¹³C (125 MHz, CDCl₃) δ 177.7 (C-2), 154.9 (C-7), 124.7 (C-6), 74.5 (C-5), 74.1 (C-8), 44.2 (C-9), 39.7 (C-11), 37.3 (C-12), 36.4 (C-3), 36.0 (C-4), 32.3 (C-13 ou C-14), 32.2 (C-13 ou C-14), 30.7 (C-15), 19.4 (C-10).

IR (film) νₘₐₓ3490, 2931, 2854, 1775, 1463, 1368, 1256, 1172, 1091, 1007, 872, 788 cm⁻¹.

HRESIMS *m*/*z* 239.1639 [M + H]⁺ (calc. pour C₁₄H₂₃O_{3,} 239.1647).

### (4 R *)-[( Z )-{ (8 R *)-(8,12,12-triméthyl-8[(triméthylsilyl)oxy]cyclohe xylidene)méthyl}] dihydrofuran-2(3 H )-one ((4 R *, 8 R *)-B23)

Un mélange d'alcool brut (4*R**, 8*R**)-B1 (94,3 mg, 0,24 mmol) et de triméthylsilylimidazole (TMS-imidazole) (1,06 mL, 7,20 mmol, 30,0 équiv.) a été agité à 50 °C sous argon pendant une nuit. Le mélange a été refroidi à température ambiante, agité pendant 1 h et dilué avec de l'heptane (5 mL). La phase organique a été lavée avec de la saumure (2 x 5 mL), séchée avec Na₂SO₄ et les solvants ont été éliminés. Le produit brut a été purifié par chromatographie sur gel de silice (heptane/EtOAc, 90:10 à 60:40 pendant 20 min) pour obtenir le produit pur (4*R**, 8*R**) -B23 de formule 11 (14,6 mg, 20% en 2 étapes) sous forme d'huile jaune :

### (4R*,8R*) -B23

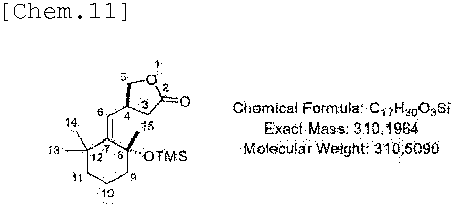

RMN ¹H (500 MHz, CDCl₃) δ 5.18 (1H, d, J = 9.5 Hz, H-6), 4.41 (1H, t, J = 8.0 Hz, H-5a), 4.31 (1H, sext, J = 9.5 Hz, H-4), 3.88 (1H, t, J = 8.0 Hz, H-5b), 2.58 (1H, dd, J = 17.0, 8.5 Hz, H-3a), 2.15 (1H, dd, J = 17.0, 8.5 Hz, H-3b), 1.86-1.81 (1H, m, H-9a), 1.74 (1H, td, J = 13.0, 4.5 Hz, H-9b), 1.59-1.53 (2H, m, H-10), 1.42 (3H, s, H-15), 1.40-1.32 (2H, m, H-11), 1.12 (3H, s, H-13 ou H-14), 1.04 (3H, s, H-13 ou H-14), 0.12 (9H, s, H-TMS).

RMN ¹³C (125 MHz, CDCl₃) δ 177.7 (C-2), 155.1 (C-7), 124.1 (C-6), 77.5 (C-8), 74.3 (C-5), 42.6 (C-9), 39.4 (C-11), 37.3 (C-12), 36.3 (C-3), 35.7 (C-4), 32.8 (C-13 ou C-14), 32.7 (C-13 ou C-14), 32.1 (C-15), 19.3 (C-10), 3.2 (C-TMS).

IR (film) νₘₐₓ 2963, 2928, 1781, 1469, 1366, 1250, 1250, 1162, 1066, 1035, 1012, 838 cm⁻¹.

HRESIMS *m*/*z* 311.1952 [M + H]⁺ (calc. pour C₁₇H₃₁O₃Si, 311.2042).

### (4R*, 8R*) -B24

À une solution de (4*R**, 8*R**)-B23 (11,2 mg, 0,04 mmol) dans du THF sec (0,4 mL) à -40 °C sous argon, est ajouté du formiate d'éthyle (32 µL, 0,4 mmol, 10,0 équiv.) et du *tert-*BuOK (32,5 mg, 0,28 mmol, 7,0 équiv.). Le mélange a été agité pendant 1 h à 0 °C. Le mélange a été dilué dans EtOAc (2 mL), lavé avec de l'eau (2 x 2 mL) et de la saumure (2 x 2 mL), séché sur Na₂SO₄ et concentré sous pression réduite pour obtenir l'énol brut (7,5 mg, 55%). Ce composé a été utilisé sans purification supplémentaire dans l'étape suivante.

A une solution d'énol brut (7,5 mg, 0,02 mmol) dans du THF sec (0,2 mL) à -78 °C ont été ajoutés du *tert*-BuOK (3,4 mg, 0,03 mmol, 1,5 équiv.) et une solution de 5bromo-3-méthylfuran-2(5H)-one D4 [12] (5,8 mg, 0,03 mmol, 1,5 équiv.) dans du THF sec (0,2 mL). Le milieu réactionel est réchauffé à température ambiante et agité pendant une nuit. Le milieu réactionnel est dilué dans EtOAc (3 mL), lavé avec de l'eau (2 x 2 mL) et de la saumure (2 x 2 mL), puis séché avec Na₂SO₄ et les solvants ont été éliminés. Le mélange a été purifié par PTLC (éther de pétrole/EtOAc, 80:20) pour obtenir le produit pur (4*R**, 8*R**)-B24 de formule 17 (2,1 mg, 12% en 2 étapes) sous forme d'huile jaune :

### (4R*, 8R*)-B24

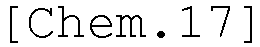

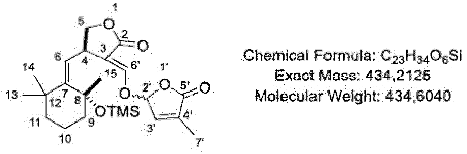

### Isomère 1 :

RMN ¹H (700 MHz, CDCl₃) δ 7.43 (1H, d, J = 3.0 Hz, H-6'), 6.79 (1H, t, J = 1.5 Hz, H-3'), 6.06 (1H, s, H-2'),5.22 (1H, dd, J = 13.5, 10.0 Hz, H-6), 4.88-4.84 (1H, m, H-4), 4.47 (1H, dd, J = 9.0, 3.0 Hz, H-5a), 3.91 (1H, sext, J = 5.5 Hz, H-5b), 1.97 (3H, t, J = 1.5 Hz, H-7'), 1.85-1.81 (1H, m, H-11a), 1.73-1.68 (1H, m, H-11b), 1.61-1.54 (2H, m, H-10), 1.46 (3H, s, H-15), 1.39-1.34 (2H, m, H-9), 1.11 (3H, s, H-13 ou H-14), 1.08 (3H, s, H-13 ou H-14), 0.13 (9H, s, H-TMS).

RMN ¹³C (175 MHz, CDCl₃) δ 172.3 (C-2), 170.4 (C-5'), 152.7 (C-7), 150.6 (C-6'), 140.9 (C-3'), 136.0 (C-4'),124.0 (C-6), 113.3 (C-3), 100.6 (C-2'), 77.7 (C-8), 72.8 (C-5), 43.0 (C-11), 39.7 (C-9), 37.3 (C-12), 37.2 (C-4), 32.8 (C-13 ou C-14), 32.7 (C-13 ou C-14), 30.9 (C-15), 19.5 (C-10), 10.9 (C-7'), 3.4 (C-TMS).

### Isomère 2 :

RMN ¹H (700 MHz, CDCl₃) δ 7.45 (1H, d, J = 2.5 Hz, H-6'), 6.83 (1H, t, J = 1.5 Hz, H-3'), 6.06 (1H, s, H-2'), 5.22 (1H, dd, J = 13.5, 10.0 Hz, H-6), 4.88-4.84 (1H, m, H-4), 4.47 (1H, dd, J = 9.0, 3.0 Hz, H-5a), 3.91 (1H, sext, J = 5.5 Hz, H-5b), 1.95 (3H, t, J = 1.5 Hz, H-7'), 1.85-1.81 (1H, m, H-11a), 1.73-1.68 (1H, m, H-11b), 1.61-1.54 (2H, m, H-10), 1.45 (3H, s, H-15), 1.39-1.34 (2H, m, H-9), 1.02 (3H, s, H-13 ou H-14),0.9 (3H, s, H-13 ou H-14), 0.12 (9H, s, H-TMS).

RMN ¹³C (175 MHz, CDCl₃) δ 172.3 (C-2), 170.4 (C-5'), 152.7 (C-7), 150.9 (C-6'), 141.0 (C-3'), 135.8 (C-4'),123.9 (C-6), 113.2 (C-3), 100.7 (C-2'), 77.6 (C-8), 72.6 (C-5), 43.1 (C-11), 39.7 (C-9), 37.3 (C-12), 37.1(C-4), 32.5 (C-13 ou C-14), 32.1 (C-13 ou C-14), 30.8 (C-15), 19.5 (C-10), 10.8 (C-7'), 3.4 (C-TMS).

IR (film) νₘₐₓ 2928, 2851, 17887, 1734, 1681, 1463, 1376, 1337, 1250, 1184, 1081, 1031, 1006, 956, 838 cm⁻¹.

HRESIMS *m*/*z* 249.1482 [M + H - H₂O]⁺ (calc. pour C₁₅H₂₁O₃, 249.1491).
**(±)-2'-epi-cannalactone et (±)-cannalactone**

À une solution de (4*R**, 8*R**)-B24 (30,1 mg, 0,070 mmol) dans CH₃CN (0,7 mL) et de l'eau (5 gouttes), on a ajouté une solution de Sc(OTf)₃ (0,3 mg, 7 µmol, 1 mol%) dans CH₃CN (0,7 mL). Le mélange résultant a été agité pendant 1 h 30 à température ambiante et hydrolysé avec un tampon phosphate (1,5 mL, pH 7). La phase organique a été extraite avec CH₂Cl₂ (3 x 2 mL), et les phases organiques combinées ont été
lavées avec de la saumure (2 x 2 mL), séchées sur Na₂SO₄, et les solvants ont été éliminés pour obtenir le produit brut. Le produit brut a été purifié par PTLC (Ether de pétrole/EtOAc, 60:40) pour obtenir le produit (±)-2'-*epi-*cannalactone (8,3 mg, 33% en 3 étapes) sous forme d'huile jaune et (±)-cannalactone de formule 5 (6,9 mg, 27% en 3 étapes) sous forme d'huile jaune.

### (±)-2'-epi-cannalactone

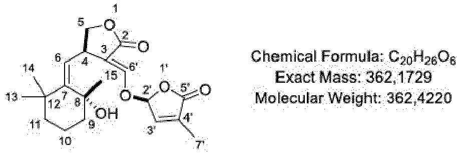

RMN ¹H (700 MHz, CDCl₃) δ 7.43 (1H, d, J = 3.0 Hz, H-6'), 6.81 (1H, t, J = 1.5 Hz, H-3'), 6.08 (1H, t, J = 1.5 Hz, H-2'), 5.32 (1H, d, J = 9.5 Hz, H-6), 4.83 (1H, tt, J = 9.0, 2.5 Hz, H-4), 4.52 (1H, t, J = 9.0 Hz, H-5a), 3.94 (1H, dd, J = 9.0, 5.5 Hz, H-5b), 1.98 (3H, t, J = 1.5 Hz, H-7'), 1.83-1.79 (1H, m, H-9a), 1.59-1.55 (2H, m, H-10), 1.53-1.48 (1H, m, H-9b), 1.43 (3H, s, H-15), 1.39-1.34 (2H, m, H-11), 1.11 (3H, s, H-13 ou H-14), 1.02 (3H, s, H-13 ou H-14).

RMN ¹³C (175 MHz, CDCl₃) δ 172.3 (C-2), 170.4 (C-5'), 152.5 (C-7), 150.5 (C-6'), 140.9 (C-3'), 136.1 (C-4'), 124.9 (C-6), 113.4 (C-3), 100.5 (C-2'), 73.9 (C-8), 73.2 (C-5), 44.6 (C-9), 40.0 (C-11), 37.3 (C-4), 37.2(C-12), 32.2 (C-13 ou C-14), 31.8 (C-13 ou C-14), 29.6 (C-15), 19.6 (C-10), 10.9 (C-7').

IR (film) νₘₐₓ 3493, 2928, 2848, 1785, 1751, 1684, 1465, 1382, 1347, 1179, 1088, 1031, 954 cm⁻¹.

HRESIMS *m*/*z* 363.1813 [M + H]⁺ (calc. pour C₂₀H₂₇O₆, 363.1808).

### (±)-cannalactone

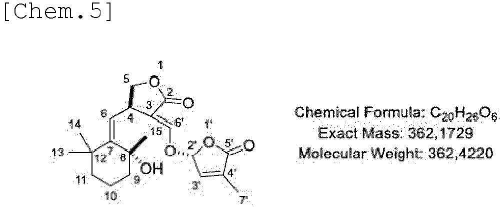

RMN ¹H (700 MHz, CDCl₃) δ 7.47 (1H, d, J = 2.5 Hz, H-6'), 6.85 (1H, t, J = 1.5 Hz, H-3'), 6.07 (1H, t, J = 1.5 Hz, H-2'), 5.31 (1H, d, J = 9.5 Hz, H-6), 4.83 (1H, tt, J = 9.0, 3.0 Hz, H-4), 4.52 (1H, t, J = 9.0 Hz, H-5a), 3.92 (1H, dd, J = 9.0, 6.0 Hz, H-5b),
1.95 (3H, t, J = 1.0 Hz, H-7'), 1.82-1.78 (1H, m, H-9a), 1.57-1.53 (2H, m, H-10), 1.51-1.47 (1H, m, H-9b), 1.43 (3H, s, H-15), 1.38-1.32 (2H, m, H-11), 1.07 (3H, s, H-13 ou H-14), 0.9 (3H, s, H-13 ou H-14).

RMN ¹³C (175 MHz, CDCl₃) δ 172.2 (C-2), 170.4 (C-5'), 152.6 (C-7), 151.1 (C-6'), 141.0 (C-3'), 135.7 (C-4'), 124.5 (C-6), 113.2 (C-3), 100.7 (C-2'), 73.8 (C-8), 72.9 (C-5), 44.6 (C-9), 39.9 (C-11), 37.3 (C-4), 37.1(C-12), 32.1 (C-13 ou C-14), 31.3 (C-13 ou C-14), 29.4 (C-15), 19.5 (C-10), 10.7 (C-7').

IR (film) νₘₐₓ 3479, 2919, 2854, 1784, 1747, 1678, 1466, 1384, 1340, 1182, 1082, 1031, 1007, 951 cm⁻¹.

HRESIMS *m*/*z* 363.1794 [M + H]⁺ (calc. pour C₂₀H₂₇O₆, 363.1808).
(+)-cannalactone [α]_{D} ²⁴ + 15.00 ± 1.59 (c 0.12, CHCl₃)
(-)-cannalactone [α]_{D}²⁴ - 11.73 ± 2.53 (c 0.13, CHCl₃)

### EXEMPLE 2 : synthèse de la cannalactone marquée au deutérium conformément au deuxième procédé selon l'invention (voie d'accès illustrée par la figure 2)

Les produits suivants sont les mêmes que préparés à l'exemple 1
- 4-bromofuran-2(5*H*)-one (C8)
- (4-bromofuran-2-yl)oxytriisopropylsilane (C4)
- 4-[hydroxy(8,12,12-triméthylcyclohex-7-èn-6-yl)méthyl]furan-2(5*H*)-one (B20)
- 4-[hydroxy(8,12,12-triméthylcyclohex-7-en-6-yl)méthyl]dihydrofuran-2(3*H*)-one (B21)
- (4*R**)-[(6*R**)-hydroxy(8,12,12-triméthyl-7-oxabicyclo[4.1.0]heptan-6-yl) méthyl] dihydrofuran-2(3*H*)-one ((4*R**, 6*R**)-*cis*-B22)
- (4*R**)-(*Z*)-[(8*R**)-hydroxy-(8,12,12-triméthylcyclohexylidene)méthyl ]dihydrofuran-2(3*H*)-one ((4*R**, 8*R**)-B1)
- (4*R**)-((*Z*)-[(8*R**)-(8,12,12-triméthyl-8{(triméthylsilyl)oxy}cyclohex ylidene)méthyl]) dihydrofuran-2(3*H*)-one ((4*R**, 8*R**)-B23).

A une solution de (4*R**, 8*R**)-B23 de formule 11 (11,0 mg, 0,035 mmol) dans du THF sec (0,35 mL) à -40 °C sous argon, on a ajouté du formiate d'éthyle (28 µL, 0,35 mmol, 10,0 équiv.) et du *tert*-BuOK (29,8 mg, 0,25 mmol, 7,0 équiv.). Le mélange a été agité pendant 1 h à 0 °C. Le mélange a été dilué dans EtOAc (2 mL), lavé avec de l'eau (2 x 2 mL) et de la saumure (2 x 2 mL), séché avec Na₂SO₄ et concentré sous pression réduite pour obtenir l'énol brut (11,4 mg, 97%).

A une solution d'énol brut (11,4 mg, 0,034 mmol) dans du THF sec (0,34 mL) à -78 °C, est ajouté du tert-BuOK (6,1 mg, 0,05 mmol, 1,5 équiv.) et une solution de composé D4 deutéré [13] (10,2 mg, 0,05 mmol, 1,5 équiv.) dans du THF sec (0,34 mL). Le milieu réactionnel est laissé réchauffé à température ambiante et agité pendant une nuit. Le mélange a été dilué dans EtOAc (3 mL), lavé avec de l'eau (2 x 2 mL) et de la saumure (2 x 2 mL), séché avec Na₂SO₄ et les solvants ont été éliminés pour
obtenir le composé brut (4*R**, 8*R**)-B24-D3 (21,2 mg) de formule 17 (alternative dans laquelle R¹ est un atome d'hydrogène et R² est le groupe CD₃) :

(4*R**, 8*R**)-B24-D3 [Chem. 17]

HRESIMS *m*/*z* 438.2393 [M + H]⁺(calc. pour C₂₃H₃₂D₃O₆, 438.2391).

A une solution de (4*R**, 8*R**)-B24-D3 (14,9 mg, 0,034 mmol) dans CH₃CN (0,34 mL) et de l'eau (5 gouttes) on a ajouté une solution de Sc(OTf)₃ (1,9 mg, 4 µmol, 10 mol%) dans CH₃CN (0,34 mL). Le mélange résultant a été agité pendant 1 h 30 à température ambiante et hydrolysé avec un tampon phosphate (1,5 ml, pH 7). La phase organique a été extraite avec CH₂Cl₂ (3 x 2 mL), et les phases organiques combinées ont été lavées avec de la saumure (2 x 2 mL), séchées avec Na₂SO₄, et les solvants ont été éliminés pour obtenir le produit brut. Le produit brut a été purifié par chromatographie sur gel de silice (éther de pétrole/EtOAc, 70:30 à 50:50 pendant 15 min) pour obtenir le produit (±)-2'-*epi*-cannalactone-D₃ (3,9 mg, 31% en 3 étapes) sous forme d'huile jaune et (±)-cannalactone-D₃ de formule 1 (alternative dans laquelle R¹ est un atome d'hydrogène et R² est le groupe CD₃) (4,1 mg, 32% en 3 étapes) sous forme d'huile jaune.

### EXEMPLE 3 : résultats biologiques

Nous avons réalisé le dosage de la cannalactone dans différentes variétés de chanvre à l'aide de la cannalactone selon l'invention selon les formules 2, 3 ou 4 selon le protocole décrit dans le pois [13]. Nous avons pu la quantifier.

### Références bibliographiques

1. Hamzaoui, O. et al., Proceedings of the 15th World Congress on Parasitic Plants; Amsterdam, The Netherlands 32, (2019).
2. Stojanova, B., Delourme, R., Duffé, P., Delavault, P. & Simier, P. Genetic differentiation and host preference reveal non-exclusive host races in the generalist parasitic weed Phelipanche ramosa. Weed Res. 59, 107-118, doi:10.1111/wre.12353 (2019).
3. Huet, S., Pouvreau, J.-B., Delage, E., Delgrange, S., Marais, C., Bahut, M., Delavault, P., Simier, P. & Poulin, L. Populations of the Parasitic Plant Phelipanche ramosa Influence Their Seed Microbiota. Front. Plant Sci. 11, 1075, doi:10.3389/fpls.2020.01075 (2020).
4. Delavault, P., Montiel, G., Brun, G., Pouvreau, J. B., Thoiron, S. & Simier, P. Communication Between Host Plants and Parasitic Plants. Adv. Bot. Res. 82, 55-82, doi:10.1016/bs.abr.2016.10.006 (2017).
5. Cook, C. E.; Whichard, L. P.; Turner, B.; Wall, M. E.; Egley, G. H.
   Germination of Witchweed (*Striga Lutea* Lour.): Isolation and Properties
   of a Potent Stimulant. Science. 154 (3753), 1189-1190. doi:10.1126/ science.154.3753.1189 (1966).
6. Daignan Fornier, S.; Keita, A.; Boyer, F.-D., Chemistry of Strigolactones, Key Players in Plant Communication. ChemBioChem doi:10.1002/ cbic.202400133 (2024).
7. Gomez-Roldan, V., Fermas, S., Brewer, P. B., Puech-Pages, V., Dun,
   E. A., Pillot, J.-P., Letisse, F., Matusova, R., Danoun, S., Portais, J.-C.,
   Bouwmeester, H., Bécard, G., Beveridge, C. A., Rameau, C. & Rochange, S. F. Strigolactone inhibition of shoot branching. Nature 455, 189-194, doi:10.1038/nature07271 (2008).
8. Umehara, M., Hanada, A., Yoshida, S., Akiyama, K., Arite, T., TakedaKamiya, N., Magome, H., Kamiya, Y., Shirasu, K., Yoneyama, K., Kyozuka, J. & Yamaguchi, S. Inhibition of shoot branching by new terpenoid plant hormones. Nature 455, 195-200, doi:10.1038/nature07272 (2008).
9. Lopez-Obando, M., Ligerot, Y., Bonhomme, S., Boyer, F.-D. & Rameau, C. Strigolactone biosynthesis and signaling in plant development. Development 142, 3615-3619, doi:10.1242/dev.120006 (2015).
10. Daignan Fornier, S. Synthèse totale de la cannalactone, strigolactone non canonique du chanvre et développement d'analogues synthétiques pour leur évaluation biologique. Université Paris-Saclay, (2023).
11. Daignan Fornier, S., de Saint Germain, A., Retailleau, P., Pillot, J.-P., Taulera, Q., Andna, L., Miesch, L., Rochange, S., Pouvreau, J.-B. & Boyer, F.-D. Noncanonical Strigolactone Analogues Highlight Selectivity for Stimulating Germination in Two Phelipanche ramosa Populations. J. Nat. Prod. 85, 1976-1992, doi:10.1021/acs.jnatprod.2c00282 (2022).
12. Macalpine, G. A., Raphael, R. A., Shaw, A., Taylor, A. W. & Wild, H. J. Synthesis of Germination Stimulant (±)-Strigol. J. Chem. Soc., Perkin Trans. 1, 410-416, doi:10.1039/C39740000834 (1976).
13. Boutet-Mercey, S., Perreau, F., Roux, A., Clavé, G., Pillot, J.-P., SchmitzAfonso, I., Touboul, D., Mouille, G., Rameau, C. & Boyer, F.-D. Validated Method for Strigolactone Quantification by Ultra High-Performance Liquid Chromatography - Electrospray Ionisation Tandem Mass Spectrometry Using Novel Deuterium Labelled Standards. Phytochem. Anal. 29, 59-68, doi:10.1002/pca.2714 (2018).
14. Jas, G. A Simple Resolution of 4-Bromo-2-(Tert-Butyldimethylsiloxy) Furan from Tetrahydro-2,4-Dioxofuran. Synthesis 11, 965-966. doi:10.1055/ s-1991-26618 (1991).

## Revendications

1. Cannalactone marquée au deutérium, **caractérisée en ce qu'**elle répond à la formule générale (1) : 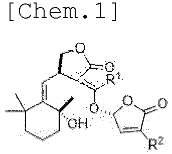 dans laquelle :
- R¹ désigne un atome d'hydrogène H ou un atome de deutérium D, - R²désigne le radical méthyle CH₃ ou le radical méthyle deutéré trisubstitué CD₃,
- l'un au moins de R¹ et R² étant deutéré.

2. Cannalactone marquée au deutérium selon la revendication 1, dans laquelle : - R¹ désigne H, et
- R² désigne CD₃.

3. Cannalactone marquée au deutérium selon la revendication 1, dans laquelle : - R¹ désigne D, et
- R² désigne CH₃.

4. Cannalactone marquée au deutérium selon la revendication 1, dans laquelle : - R¹ désigne D, et
- R² désigne CD₃.

5. Procédé de synthèse de la cannalactone, de formule générale (5) [Chem.5] 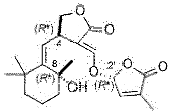 **caractérisé en ce qu'**il comprend les étapes suivantes :
- une réaction A) de couplage du β-cyclocitral commercial avec unbromofurane C4 de formule (6) 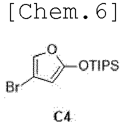 pour obtenir un alcool B20 de formule (7) [Chem.7] 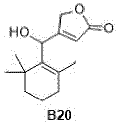
- une étape B) de réduction de l'alcool B20 de formule (7), pour obtenir un mélange de diastéréoisomères de l'alcool allylique, suivie d'une étape de séparation, desdits diastéréoisomères pour retenir le diastéréoisomère (*4R*, 6R**)-B21 de formule (8) [Chem.8] 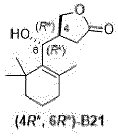
- une étape C) d'époxydation sélective du composé (*4R*, 6R**)-B21 de formule (8) pour obtenir l'époxyde *4R*, 6R**)*-cis-*B22 de formule (9) 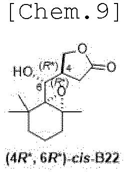
- une réaction radicalaire D) de réduction et d'isomérisation de l'époxyde (*4R*, 6R**)*-cis-*B22 de formule (9), pour obtenir l'alcool tertiaire *(4R*,* 8R*)-B1 de formule (10) [Chem.10] 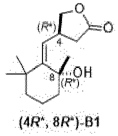
- une étape E) de protection de l'alcool tertiaire (*4R*, 8R**)*-* B1 de formule (10), pour obtenir l'alcool protégé (*4R*,* 8*R**)-B23 de formule (11) [Chem.11] 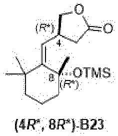
- une étape F) comprenant la formylation F1) du composé (*4R**, *8R**)-B23 de formule (11) à l'aide de formiate d'alkyle répondant à la formule (12) [Chem.12] 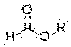 puis l'*O*-alkylation F2) de l'aldéhyde ainsi formé à l'aide du composé D4 de formule (13) 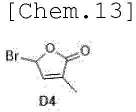 conduisant à l'obtention du mélange de diastéréoisomères (4*R**,6*R**)B24 de formule (14) 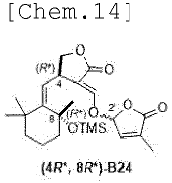
- une étape G) de déprotection et de séparation des diastéréoisomèresde formule (14), conduisant à l'isolement de la cannalactone de formule (5).

6. Procédé de synthèse d'une cannalactone marquée au deutérium telle que définie selon l'une quelconque des revendications 1 à 4, **caractérisée en ce qu'**il comprend les étapes suivantes :
- les étapes A à E de synthèse du composé protégé (*4R**, *8R**)-B23 de formule (11) telles que définies à la revendication 5 ;
- une étape F) comprenant la formylation F1) du composé (4*R**, 8*R** )-B23 de formule (11) à l'aide du formiate d'éthyle répondant à la formule (12)
ou
à l'aide du formiate d'alkyle deutéré répondant à la formule (15) [Chem.15] 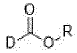
- puis l'*O*-alkylation de l'aldéhyde ainsi formé à l'aide du composé D4 de formule (13) 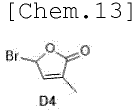 ou à l'aide du composé D4 deutéré de formule 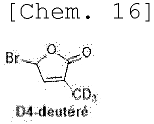 l'un au moins du composé organique fonctionnalisé par un groupe formyle ou du composé D4 étant deutéré, pour obtenir un mélange de diastéréoisomères deutérés (4*R**,6*R**)B24 de formule (17) 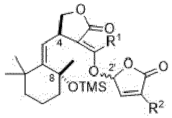 avec
- R¹ désignant un atome d'hydrogène H ou un atome de deutérium D, et
- R² désigne un groupement méthyle (CH₃) ou un groupement méthyle où les hydrogènes ont été remplacés par des atomes de deutérium (CD₃) ;
- une étape G) de déprotection et de séparation des diastéréoisomères, conduisant à l'isolement d'une cannalactone marquée au deutérium de formule (1).

7. Procédé selon la revendication 6, dans lequel :
- la formylation F1) est réalisée du formiate d'alkyle répondant à la formule (12) ; et
- l'O-alkylation F2) de l'aldéhyde à l'issue de la formylation F1) est réalisée à l'aide du composé D4 de formule 16.

8. Procédé selon la revendication 6, dans lequel :
- la formylation F1) est réalisée à l'aide du formiate d'alkyle deutérérépondant à la formule (15) ; et
- l'*O*-alkylation F2) de l'aldéhyde à l'issue de la formylation F1) est réalisée à l'aide du composé D4 de formule (13).

9. Procédé selon la revendication 6, dans lequel :
- la formylation F1) est réalisée à l'aide du composé organique fonctionnalisé par un groupe formyle répondant à la formule (15) ; et
- l'*O*-alkylation F2) de l'aldéhyde à l'issue de la formylation F1) est réalisée à l'aide du composé D4 de formule (16).

10. Utilisation de la cannalactone marquée au deutérium telle que définie selon l'une quelconque des revendications 1 à 4 ou telle qu'obtenue selon l'une quelconque des revendications 6 à 9, pour effectuer le dosage de la cannalactone dans tous tissus ou exsudats de plantes ou organismes vivants. Et notamment dans les exsudats ou tissus de la plante de chanvre.

11. Utilisation selon la revendication 10, pour effectuer le dosage de la cannalactone naturelle des exsudats ou tissus de la plante de chanvre par chromatographie liquide couplée à la spectrométrie de masse.
